(19) Europäisches Patentamt
European Patent Office
Office européen des brevets

(11) Publication number: **0 495 366 A2**

# (12) EUROPEAN PATENT APPLICATION

(21) Application number: **92100111.1**

(22) Date of filing: **07.01.92**

(51) Int. Cl.[5]: **C12Q 1/54**, //G01N33/52, C12Q1/00

(30) Priority: **14.01.91 US 641172**

(43) Date of publication of application: **22.07.92 Bulletin 92/30**

(84) Designated Contracting States: **DE FR GB IT**

(71) Applicant: **MILES INC.**
**Mobay Road**
**Pittsburgh, PA 15205-9741(US)**

(72) Inventor: **Greene, Carmine M.**
**52996 Highland Drive**
**South Bend, Indiana 46635(US)**
Inventor: **Wu, Wen H.**
**51819 Winding Waters Lane**
**Elkhart, Indiana 46514(US)**

(74) Representative: **Dänner, Klaus, Dr. et al**
**c/o Bayer AG Konzernverwaltung RP Patente Konzern**
**W-5090 Leverkusen 1 Bayerwerk(DE)**

## (54) Ascorbate resistant composition and test device for detecting a component in a liquid test sample.

(57) A dip-and-read type indicator as described which is capable of detecting the presence of a component in a test solution, despite the presence of an interfering substance. The invention particularly directed to the detection of glucose in urine samples in the presence of a reducing agent, such as ascorbic acid. The invention uses mercury (II) oxide complexes with various ligands such as alanine, lysine, Tris/glutamate, Tris/succinate, DL-$\beta$-Aminobutyric acid, and DL-$\gamma$-Aminobutyric acid. These ligands were found to be suitable because of their ability to complex with HgO, their ability to chemically react with the interfering substance, and their ability to control the pH of the dip solution.

EP 0 495 366 A2

## FIELD OF THE INVENTION

The present invention relates to minimizing the interfering effects of certain reducing agents on the analysis of a component in a liquid test solution containing one or more of these interfering reducing agents.

The art of analytical chemistry has been greatly advanced since biochemistry began emerging as a primary scientific frontier requiring increasingly sophisticated analytical methods and tools to solve problems, the solutions of which were never before attempted. Likewise, the medical profession has lent impetus to the growth of analytical chemistry, requiring both high precision and speed in obtaining results. This remarkable progress has been still further spurred by industries such as brewing, chemical manufacturing, and others.

To satisfy the needs of these expanding technologies, a myriad of analytical procedures, compositions and apparatuses have evolved, including solution chemistry techniques, automated machinery and the so-called "dip-and-read" reagent strips. It is to the last of these that the present invention is primarily directed, although substantial benefit ultimately inures to the other procedures as well.

Reagent strip type test devices enjoy wide use in many analytical applications, especially in the chemical analysis of biological fluids, because of their relative low cost, ease of utilization and speed in obtaining results. In medicine, for example, numerous physiological functions can be monitored merely by dipping reagent strips into a sample of body fluid, such as urine, and observing a detectable response such as a change in color or a change in the amount of light reflected from or absorbed by the strip.

Compatible with such "dip-and-read" methods, there have arisen many chemistries for detecting body fluid components. Most of these produce a detectable response which is quantitative or at least semiquantitative. Thus, by measuring the response after a predetermined time, the analyst can obtain not only a positive indication of the presence of a particular constituent in a test sample, but also an estimate of how much of the constituent is present. Such strips provide the physician with a facile diagnostic tool as well as the ability to gauge the extent of disease or bodily malfunction.

Illustrative of dip-and-read strips currently in use are products available from the Ames Division of Miles, Inc. under the trademarks CLINISTIX^R, MULTISTIX^R, KETOSTIX^R, N-MULTISTIX^R C, DIASTIX^R, DEXTROSTIX^R, and others. Test devices such as these usually comprise one or more carrier matrices, such as absorbent paper, having respectively incorporated with them a particular reactant system which manifests a color change in the presence of a specific test sample components. Depending on the reactant system incorporated with a particular matrix, these devices can detect the presence of glucose, ketone bodies, bilirubin, occult blood, nitrite, and other pathological substances. The specific color change and the intensity of the color observable within a specific time range after contacting the strip with the sample is indicative of the presence of a particular component and its concentration in the sample. Some of these test devices and their reactant systems are set forth in U.S. Pat. Nos. 3,123,443 (CLINISTIX^R); 3,212,855 and 4,147,514 (KETOSTIX^R); 3,814,668, 3,164,534 and 2,981,606 (DIASTIX^R); and 3,298,789, 3,092,465, 3,164,534 and 2,981,606 (DEXTROSTIX^R).

The reagents which change color to indicate the presence of a substance in body fluids have been fully described in the prior art, such as U.S. Patent No. 4,288,541. While a complete discussion of these reagents is not included herein, because of its inclusion of U.S. Patent No. 4,288,541, only the basic background of these reagents will be reviewed below.

Because of the relatively high incidence of diabetes mellitus and its accompanying serious clinical consequences, high interest from the biological and medical professions arose in new techniques for analyzing glucose levels in urine and serum. This keen interest led to the development of several procedures which deviate dramatically from their solution chemistry forbears. These utilize sophisticated biochemical systems which can be incorporated into dry, dip-and-read devices, used in solution or suspension techniques, or in conjunction with spectrophotometers and other hardware.

Of these new techniques, the present invention lends itself especially well to an enzymatic system wherein the analyte, for instance glucose, is a substrate for a particular enzyme, the reaction products being capable of eliciting a detectable response for chromogenic indicator compounds, such as those known loosely in the art as "benzidine-type indicators". These will be more carefully defined, infra, but for the present, suffice it to say these compounds can undergo color changes in the presence of hydrogen peroxide and a peroxidative substance, such as the enzyme peroxidase. The glucose/glucose oxidase system exemplifies the prior art, wherein glucose is oxidized to gluconic acid with the concomitant formation of $H_2O_2$ in accordance with:

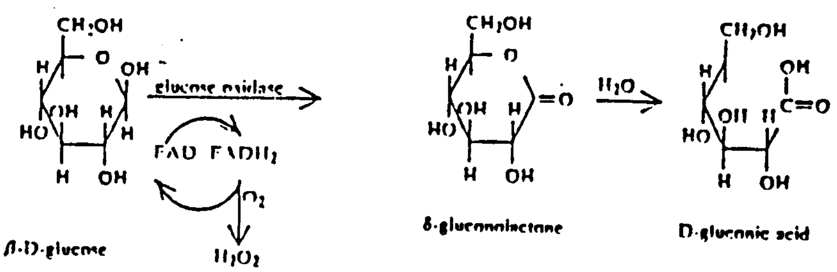


β-D-glucose δ-gluconolactone D-gluconic acid

It is the formation of hydrogen peroxide which facilitates the subsequent, indicator-related steps leading to observable color formation or other detectable response. Thus a benzidine-type indicator responds in the presence of hydrogen peroxide and peroxidase by changing its light absorptive capability.

In practice, this technology is presently utilized for glucose analysis in the form of dip-and-read reagent strips such as those marketed by the Ames Division of Miles, Inc. under the trademark CLINISTIX$^R$ and others. Broadly, these comprise a plastic strip, at one end of which is mounted an absorbent paper portion impregnated with the appropriate enzymes, indicator compound and buffering agents as the principal active ingredients. They are used by dipping the reagent-bearing end into the test sample, removing it and comparing any color formed in the paper with a standard color chart calibrated to various glucose concentrations.

Despite the remarkable gains provided by the reagent strips, certain substances often present in the test sample are often found to interfere with the accuracy of the test. When the concentrations of such substances reach a certain threshold level, in comparison to that of the substrate measured, the adverse effect on the test can become marked. For example, those skilled in the art of reagent strips have for a long time been aware that the presence of ascorbic acid in urine can adversely affect the analysis of such nonrelated components as glucose, occult blood, bilirubin, and nitrite. Thus, high urinary concentration of ascorbic acid from therapeutic doses of vitamin C or parenteral preparations which contain vitamin C as a reducing agent; e.g., tetracyclines, can inhibit the reaction of such tests and limit their accuracy.

Such color changing reagents may also be used in accordance with the invention described hereinbelow.

## DESCRIPTION OF THE PRIOR ART

A description of the prior art can be found in the disclosure of the Magers et al. Patent No. 4,288,541. The inventors in that patent sought to improve on the prior art devices by providing a specific class of ligands to complex with the $Hg^{++}$ ion. The ligand bonded covalently with the mercuric ion and formed a water-soluble complex. The complex was also found to have a relatively high oxidation potential.

U.S. Patent No. 4,288,541 discloses the use of an HgO/Sarcosinate complex, and found this complex to be especially suited for detection of glucose in the presence of interfering reducing agents. Ascorbic acid is one example of a reducing agent that is often found in body fluids, such as urine. It has been found that HgO/Sarcosinate complex suffers from the following disadvantages:

a) Low stability of complex;
b) Little buffer capacity; and
c) Difficulty in controlling the pH of the dip solution.

The pH of the dip solution cannot be adjusted using the HgO/Sarcosinate complex. The pH of this complex is 6.5. In many cases, when the HgO/Sarcosinate second dip was applied, the strips were not adequately reactive. When strips were found to be unreactive, another dip into a solution with a pH different from 6.5 was required.

## OBJECTIVES OF THE INVENTION

It is an objective of this invention to provide a dip-and-read type reagent test strip device that is able to detect the presence of a compound of interest in the presence of an interfering substance.

It is a further objective of this invention to provide a complex for such a dip-and-read type reagent test strip device wherein the pH of the dip solution may be controlled by the particular complex chosen.

It is a further objective of this invention to overcome the disadvantages of the prior art.

Briefly stated, the present invention is based upon an improved composition capable of detecting the

presence of a component in a liquid test sample. The prior art composition, which Applicants have improved, comprises a reagent system capable of producing a detectable response, such as a color change, in the presence of the component, and a reducing agent trapping system comprising a compound of heavy metal ion having an oxidation potential between that of the chromogenic substance and that of reducing agent having an oxidation potential similar to that of ascorbic acid. This composition has been dramatically improved by Applicants by providing the metal ion as a complex $Hg^{++}$ and a specific class of ligands. The ligands are specially chosen to be able to control the dip solution pH, to react with the reducing agent to prevent interference with the detection, and to provide adequate mercury oxide solubility. Examples of the ligands which may be used to practice this invention include: alanine, lysine, Tris/Glutamate, Tris/Succinate, DL-$\beta$-Aminobutyric acid, and $\gamma$-Aminobutyric acid. These ligands have been found to be soluble with HgO, and thus able to form a complex with HgO. The ligands may also be used to control the pH of the dip solution, and the ligands are also effective at oxidizing the interfering reducing agent.

The invention additionally comprises a test device made by incorporating the composition with a carrier matrix, as well as a method for analysis utilizing the composition or device by contacting either with the test sample and observing a detectable response.

## BRIEF DESCRIPTION OF THE DRAWINGS

These and other advantageous aspects of this invention will be more fully appreciated from the following detailed description and the attached figures. The figures include:

FIG. 1 which shows the spectral reflectance data of an HgO/Tris-glutamate indicator with no ascorbate in the solution to be tested;

FIG. 2 shows the spectral data of the same indicator as Fig. 1, with 50 mg% (milligrams per deciliter) ascorbate in the solution;

FIG. 3 shows a composite of Figs. 1 and 2 comparing the spectral data for HgO/Tris-glutamate;

FIG. 4 shows the spectral reflectance data of an HgO/Tris-succinate indicator with no ascorbate in the solution to be tested;

FIG. 5 shows the spectral data of the same indicator as Fig. 4, with 50 mg% ascorbate in the solution;

FIG. 6 is a composite graph of Figs. 4 and 5, comparing the spectral data at a 1% glucose concentration; and

FIG. 7 shows a composite of Figs. 1 and 2 comparing the spectral data of the control solution, HgO/Trissarcosinate.

## DETAILED DESCRIPTION OF THE PREFERRED EMBODIMENTS

The use of the mercuric ion/ligand complex, in accordance with the present invention, produces test strips which can detect glucose in urine, in the presence of reducing agents, such as ascorbic acid. Even when the test solutions contain relatively high amounts of reducing agents, analysis is still possible. For example, the complexes of the present invention have been found to facilitate accurate analysis of glucose in urine containing in excess of 50 milligrams per deciliter (mg%) of ascorbate. Yet the compositions are stable and may be stored for relatively long periods of time.

The complexes of this invention have the additional advantage over the complexes of U.S. Patent 4,288,541, in that by varying the concentration of the Tris to suit the requirements of the system, these new complexes may further be used to control the pH of the solutions containing the complex.

One chemical used in U.S. patent 4,288,541 consists of a complex of HgO/Sarcosinate. The inventors in that patent found that this complex is well suited for detection of glucose in urine, in the presence of interfering reducing agents, such as ascorbic acid. This complex further suffers from the following disadvantages: Sarcosinate is not very stable, it has little buffer capacity, and it may not be used to control the pH of the dip solutions. With the sarcosinate based complexes, it is impossible to predetermine what the pH of the final solution would be.

The sarcosinate complex has a pH of 6.5. When the sarcosinate solution is made, it is not possible to control the pH of the dip solution. In some instances, when the HgO/Sarcosinate second dip is applied, the test strips are not adequately reactive. Another dip, with a pH different from 6.5, is then required to make the strips reactive. This additional dip increases the costs of producing the strips and increases the production time. There is also a danger that some non-reactive strips will be manufactured and released to the public.

Several ligands were found that may be used to practice this invention. Mercury (II) oxide is complexed

with one of the following ligands: Tris-glutamate, Tris-succinate, alanine, lysine, DL-β-Aminobutyric acid and γ-Aminobutyric acid. It was found that these ligands were effective in dissolving mercury (II) oxide and thus forming the complex with mercury oxide. These ligands were also successful at controlling the dip solution pH and showed an adequate rate of oxidation of ascorbic acid. It was also found that DL-β-Aminobutyric acid and γ-Aminobutyric acid were successful ligands, although these two chemicals did not display the high solubility for HgO that the other chemicals do. This data is summarized in Table I.

### TABLE I

SOLUBILITY OF HgO IN THE SOLUTION OF VARIOUS LIGANDS

| Ligand | Chemical Structure | HgO Solubility Index | Solution pH | TRate of Ascorbate Oxidation |
|---|---|---|---|---|
| Alanine | 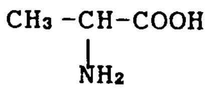 | 5+ | 6.30 | <15 sec. |
| Lysine | 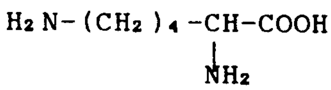 | 5+ | 10.43 adj. to 6.05 | <15 sec. |
| Tris/Glutamate | (Tris) $(CH_2OH)_3CNH_2$ | 3+ | 6.84 adj. to 5.53 | <15 sec. |
| | (Glutamate) 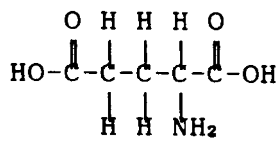 | | | |
| Tris/Succinate | (Tris) $(CH_2OH)_3CNH_2$ | 5+ | 4.6 | <15 sec. |
| | (Succinate) 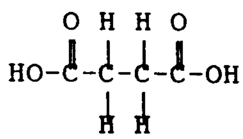 | | | |
| DL-β-Aminobutyric acid | 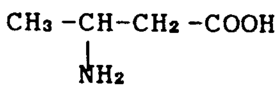 | 1+ | 7.89 adj. to 5.50 | <15 sec. |
| γ-Aminobutyric acid | 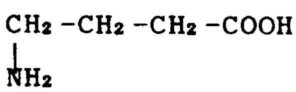 | 1± partially soluble gray ppt | 7.46 | <15 sec. |
| Glycine | $H_2N-CH_2-COOH$ | 0 | -- | -- |

The table shows the degree of solubility of HgO in the solution of the various ligands. The solubility tests were carried out as follows:

0.25 g HgO (yellow color) from Aldrich Chemical Co., Milwaukee, Wisconsin and 5 mmoles of each ligand are dissolved in 5 ml water. The HgO used may be either Aldrich 22, 108-2 (yellow, 99 + % HgO) or Aldrich 27,636-7 (yellow, 98% Hg).

The HgO solubility index key is as follows:

O = Insoluble

1± = Partially soluble, turbid suspension
1+ = Soluble overnight, clear final solution
3+ = Soluble within 1 hour, clear final solution
5+ = Soluble within 10 min., clear final solution.

The ascorbic acid oxidation rate in Table I was tested as follows:

100 µl of HgO/ligand complex was added to 1 ml of 100 mg/dl (mg%) ascorbic acid solution in 0.1 M of citrate buffer, pH = 5.5. The rate of ascorbic acid oxidation was measured by testing with a C-Stix$^R$. The C-Stix$^R$ is a dip and read reagent strip with an active ingredient of sodium phospho-12-molybdate, and is manufactured by Miles, Inc. The time required for the disappearance of ascorbic acid was measured.

A general formula for the ligands which the inventors have found to form a stable complex with HgO is as follows:

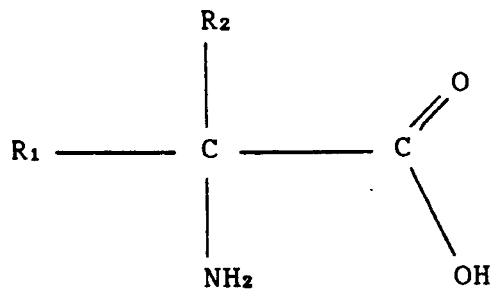

FORMULA A

This formula includes α-amino acids with various side chains for $R_1$ and $R_2$. Typically, $R_2$ is hydrogen and $R_1$ is a methyl group, as in alanine. Alanine has been found as a successful ligand by the inventors.

However, when both $R_1$ and $R_2$ are hydrogen, thus forming glycine, it has been found that this compound is not a successful ligand, because it would not dissolve HgO (see Table I).

It is believed that the nature of the $R_1$ branch in formula A is at least partially responsible for determining the solubility of the HgO ligand complex. When $R_1$ is an alkyl group with four carbon atoms, thus forming leucine, the resulting complex is not soluble. However, when an amino acid group is attached to the four carbon alkyl chain, as in lysine, the resultant complex is soluble.

Guanido groups, forming arginine were found only to provide partial solubility. Sulfhydryl derivatives as in cysteine or methionine were found to be readily oxidized by HgO and thus formed gray precipitates.

Formula A also extends to include β-amino acids, such as β-aminobutyric acid, as shown in Table I. The solubility of the complex is shown to be somewhat reduced for the β-aminobutyric acid sample in Table I, as compared to the other ligands; however, the DL-β-Aminobutryic acid does improve the degree of solubility of HgO to some extent, and is therefore considered a successful ligand.

Complexes which form γ-amino acids, such as γ-aminobutyric acid also improved the solubility of HgO to some extent, and are therefore considered successful ligands.

A second class of effective ligands for HgO solubilization include amino-alcohols as depicted by the following formula:

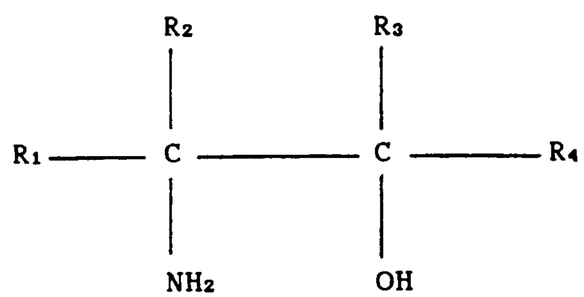

FORMULA B

Tris(hydroxymethyl)aminomethane (TRIS), for example, was found to be an effective solubilizer. Glucosamine was also found to be an excellent solubilizer, as the complex went into solution in less than ten minutes. Glucosamine was not stable against HgO oxidation and a gray precipitate formed after 5 hours.

It is noted that U.S. Patent 4,288,541 lists alanine and lysine as unsuccessful ligands in Tables II and III. That patent mentions that the alanine formed metallic mercury upon aging and lysine formed a gray precipitate (see Table III). It has now been determined that alanine and lysine are successful ligands.

It is believed that this apparent inconsistency may be explained. Note that in Examples XII-XXXVI in Patent No. 4,288,541, the mercury concentration was 0.845 moles/liter. The present invention has a substantially lower mercury concentration of 0.230 moles/liter. It is believed that this lower mercury concentration in the instant invention is the reason that alanine and lysine have now been found to be successful when they were previously listed as unsuccessful in the Magers patent.

In preparing the test device of the present invention, wherein the composition is the glucose-responsive reagent described, supra, the composition comprises a glucose-responsive reagent solution in water (first solution), and a solution containing the mercury complex (second solution). The glucose-responsive solution contains a benzidine-type indicator such as 3, 3', 5, 5'-tetramethylbenzidine, glucose oxidase, peroxidase, and buffer. A piece of filter paper is immersed and saturated with the first solution and dried. Next the dried impregnated filter paper is immersed and saturated with the second solution and dried.

In one embodiment of the invention, the paper containing the first and second solution reagents is cut into small squares, one of which is mounted at one end of a strip of polystyrene film. Adhesion of the paper to the polystyrene can be effected using a double-faced adhesive tape such as that known as Double-Stick$^R$ marketed by 3M Co. The resultant test device can then be used to measure glucose in urine, the test being virtually uninhibited by the presence of 50 mg% ascorbic acid or more in the test sample.

The carrier matrix utilized in this invention can comprise any substance capable of being incorporated with the composition. Thus the matrix can take on many known forms, such as those utilized for reagent strips for solution analysis. A porous material as the carrier matrix is preferred. For example, U.S. Patent No. 3,846,247 teaches the use of felt, porous ceramic strips, and woven or matted glass fibers. As substitute for paper, U.S. Patent No. 3,552,928 teaches the use of wood sticks, cloth, sponge material, and argillaceous substances. The use of synthetic resin fleeces and glass fiber felts in place of paper is suggested in British Patent No. 1,369,139. Another British Patent No. 1,349,623, suggests the use of a light-permeable meshwork of thin filaments as a cover for an underlying paper matrix. This reference also suggests impregnating the paper with part of a reagent system and impregnating the meshwork with other potentially incompatible reagents. French Patent No. 2,170,397 teaches the use of carrier matrices having greater than 50% polyamide fibers therein. Another approach to carrier matrices is disclosed in U.S. Patent No. 4,046,513 wherein the concept of printing reagents onto a suitable carrier matrix is employed. U.S. Patent No. 4,046,514 discloses the inter-weaving or knitting of filaments bearing reagents in a reactant system. Preferably the carrier matrix comprises a bibulous material such as filter paper. All such carrier matrix concepts can be employed in the present invention, as can other carriers which are known to those skilled in the art.

The base support member on which the impregnated carrier matrix can be mounted may take on many variations in shape, size and material of construction. Thus, it might be constructed of any substantially liquid impervious material, such as polystyrene, polyolefin, glass, paper, metal or other material. Usually, however, it is preferred that the base member of a polymeric material, such as biaxially oriented polystyrene sold by Plastic Suppliers, Inc. For most purposes it has been found preferable that the support member be relatively rigid and extend sufficiently far from the carrier matrix position to afford the user a convenient handle.

The following is an example of a first preferred embodiment of the complex of the invention. The final product is a complex of HgO/Tris/glutamate.

EXAMPLE 1

A number of test strips were prepared using glucose-sensitive reagents and mercuric ligands. Each was prepared in the form of an oblong polystyrene strip, on one end of which was mounted a square of filter paper impregnated with the composition. The paper was held in place using double-faced adhesive tape.

In preparing these test devices, a 0,5 cm (0.2 inch) wide strip of Whatman 54 Filter Paper was immersed in a first dip solution, described more fully below, containing the color changing reagent, the solution comprising glucose oxidase, peroxidase, 3,3',5,5'tetramethyl-benzidine, and a buffer in water. The impregnated paper was then dried in an air oven at about 60 ° C for 25 minutes. Following drying, the paper was next immersed in a second dip solution, described more fully below, comprising the mercuric ligand

complex. After a second drying, the impregnated strip was mounted along one edge of a film of biaxially oriented polystyrene using a double-faced adhesive known as Double Stick[R], marketed by 3M Company. The filter paper/film composite was cut in strips perpendicular to the edge bearing the impregnated paper. The strips measured about 4 x 0,5 cm (0.2 inches), the paper portion at the ends each measuring about 0,5 cm (0.2 inches) square.

The first dip solution contained the ingredients listed below. These were mixed in order as listed.

| 1st Dip: | |
|---|---|
| 2M Tris-hydroxymethylaminomethane | 50 millimoles (mM) per liter |
| Succinic Acid | 350 mM |
| Polyvinylpyrrolidone (K-60, obtained from GAF Corp.) | 3 g% by weight |
| Ascorbic acid | 30 mg% by weight |
| Water | |
| Acetone | 35% |
| 3, 3', 5, 5'-Tetramethyl-benzidine | 20 mM |
| Gantrez ES225 (GAF Corp.) | 3 g% |
| 1M Mercaptosuccinate | 7 mM |
| Sarkosyl NL-30 (Ciba-Geigy Corp.) | 80 mg% |
| Isopropanol | 18% |
| 5M KOH | 70 mM |
| Glucose oxidase (Sigma Chemical Co., 145,000 units per gram solid) | 160 units/ml |
| Horseradish peroxidase (Miles Inc., 126 units per milligram solid) | 290 units/ml |

The second dip solution was prepared by first mixing Tris-hydroxymethylaminomethane, glutamic acid, and HgO in water. The other ingredients were then added to give these concentrations:

| 2nd Dip: | |
|---|---|
| Tris-hydroxymethylaminomethane | 250 mM |
| Glutamic Acid | 250 mM |
| HgO | 100 mM |
| Polyvinylpyrrolidone (K-60, obtained from GAF Corp.) | 3 g% |
| Emulphor ON-870, (GAF Corp.) | 45 mg% |
| Isopropanol | 10 % |
| Water | |

The papers were made with Whatman 54 paper. They were dried at 60°C for 25 minutes.

In Figures 1 and 2 the reactivity of the reagent strip containing an HgO/sarcosinate complex (control solution) was compared to the reactivity of the reagent strip containing the Hgo/Tris/glutamate complex, in accordance with this invention. A reflectance spectra for the HgO/sarcosinate complex control solution with and without ascorbic acid is shown in Fig. 7. Figures 1 and 2 show the reflectance spectra for these test strips. Figure 1 is a 3% glucose solution with no ascorbic acid present. Notice that the curve marked "X" is for a HgO/sarcosinate complex solution which was used as a control solution, and the curve mark "O" is for the HgO/Tris/glutamate complex solution in accordance with the invention. The preparation of the HgO/sarcosinate control solution is the same as the preparation of the HgO/Tris/glutamate solution, except that 200 mmoles of sarcosinate is substituted for the 250 mM/l Tris-hydroxymethylaminomethane and 250 mM/l glutamic acid in the second dip. The reflectance data was collected on a Rapid Scanning Reflectance Spectrophotometer. The construction and operation of the Rapid Scanning Reflectance Spectrophotometer is fully described in an article entitled "Rapid Scanning Reflectance Spectrophotometer", by Genshaw, M.A. and Rogers, R.W., Analytical Chemistry, Vol. 53, 1981, pgs. 1949-1952, which article is incorporated herein by reference.

Figure 2 shows the spectra obtained from a 3% glucose solution with 50 mg% ascorbate present in the test solution. By comparing spectra in Figures 1 and 2, note that reagent strips containing HgO/Tris/glutamate effectively eliminate interference by the reducing agent ascorbic acid. The curves in Figure 2 are labelled the same as those in Figure 1.

Figure 3 is a composite graph of the data for HgO/TRIS/glutamate in Figures 1 and 2, with 3 g/dl urine

glucose with and without 50 mg/dl ascorbic acid present. Note that there is only a slight change in the Reflectance Spectra for sample solutions which contain no ascorbate (represented by the open box of Fig. 3), and the solutions which contain 50 mg% ascorbate (represented by the blackened in box in Fig. 3). This composite graph shows that the test strips of the invention may be used to determine the presence of glucose, despite the presence of the strong reducing agent, i.e., ascorbate. Figure 7 is a similar composite graph for the HgO/Tris/sarcosinate control solution, with a 3 g/dl urine glucose standard with and without 50 mg/dl ascorbic acid present.

The next example shows a second embodiment of the invention which uses an HgO/Tris/succinate complex.

## EXAMPLE 2

This test strips for this Example were prepared in the same manner as Example 1, the first dip solution contained the ingredients listed below, mixed in the order as listed:

Strips made with HgO/Tris/succinate

| 1st Dip: | |
|---|---|
| 2M Tris-hydroxymethylaminomethane | 50 mmoles (mM) per liter |
| Succinic Acid | 150 mM |
| Polyvinylpyrrolidone (K-60, obtained from GAF Corp.) | 3 g% by weight |
| Ascorbic acid | 30 mg% by weight |
| Water | |
| Acetone | 35% |
| 3, 3', 5, 5'-Tetramethylbenzidine | 20 mM |
| Gantrez ES225 (GAF Corp.) | 3 g% |
| 1M Mercaptosuccinate | 7 mM |
| Sarkosyl NL-30 (Ciba-Geigy Corp.) | 80 mg% |
| Isopropanol | 18% |
| 5M KOH | 70 mM |
| Glucose oxidase in water (Sigma Chemical Co., 145,000 units per gram solid) | 160 units/ml |
| Horseradish peroxidase in water (Miles Inc.,126 units per milligram solid) | 290 units/ml |

The second dip was prepared by first mixing Tris/hydroxymethylaminomethane, succinic acid, and HgO in water.

The other ingredients were then added to give these concentrations:

| 2nd Dip: | |
|---|---|
| Tris-hydroxymethylaminomethane | 330 mM |
| Succinic Acid | 250 mM |
| HgO | 100 mM |
| Polyvinylpyrrolidone (K-60, obtained from GAF Corp.) | 3 g% |
| Emulphor ON-870 (GAF Corp.) | 50 mg% |
| Water | |

The papers were made with Whatman 54 paper. They were dried at 60° C for 25 minutes.

Figures 4 and 5 show the spectral data for the HgO/Tris/succinate complex of Example 2. Figure 4 is the spectral data with no ascorbate added to the solution. Figure 5 shows the spectral data with 50 mg% of the reducing agent ascorbate in the solution. The graphs of Figures 4 and 5 show the spectral data for a variety of glucose concentrations. The concentrations used in the graphs are 1%, 2%, 4% and 8% glucose, with the curves labelled 1, 2, 4, and 8, respectively.

Figure 6 is a composite graph which shows a comparison of the data of Figures 4 and 5 with a 1% glucose solution (i.e., lg/dl urine glucose standard with and without 50 mg/dl ascorbic acid present). Note

that Figure 6 shows virtually no change in the spectral data for the HgO/Tris/succinate complex in spite of the presence of a strong reducing agent. Again, the open boxes represent the test wherein ascorbic acid is not present, and the blackened boxes represent the test with 50 mg/dl ascorbic acid is present.

While the invention has been described with particular examples and embodiments, various changes and modifications may be made without departing from the spirit and scope of the invention, as defined in the following claims.

## Claims

**1.** An indicator for detecting the presence of a component in a test solution, wherein said test solution may further include an interfering substance the indicator comprising:

a reagent which chemically reacts with the component in the test solution, if any of the component is present in the test solution, whereby the reagent indicates the presence of the component by a change in color or by a change in its light reflectance or absorption spectra;

an HgO/ligand complex, said complex formed from a complex solution including the HgO and the ligand;

said ligand of the complex being chosen such that the ligand dissolves the HgO, thus forming said HgO ligand complex, and the Hgo/ligand complex chemically reacting with any present interfering substance, thus eliminating any adverse effects of the interfering substance, and the Hgo/ligand complex being used to control the pH of the complex solution.

**2.** An indicator as claimed in claim 1, wherein the component is glucose.

**3.** An indicator as claimed in any of claims 1 and 2, wherein the interfering substance is a reducing agent.

**4.** An indicator as claimed in claim 3, wherein the reducing agent is ascorbic acid.

**5.** An indicator as claimed in any of claims 1 to 4, wherein the complex is selected from the group HgO/Tris/glutamate, HgO/Tris-succinate, HgO/Alanine or HgO/DL-$\beta$-Aminobutyric acid.

**6.** An indicator as claimed in any of claims 1 to 5, wherein the molar ratio of ligand to HgO is at least 4.

**7.** Use of the indicator according to any of claims 1 to 6 in determination of glucose.

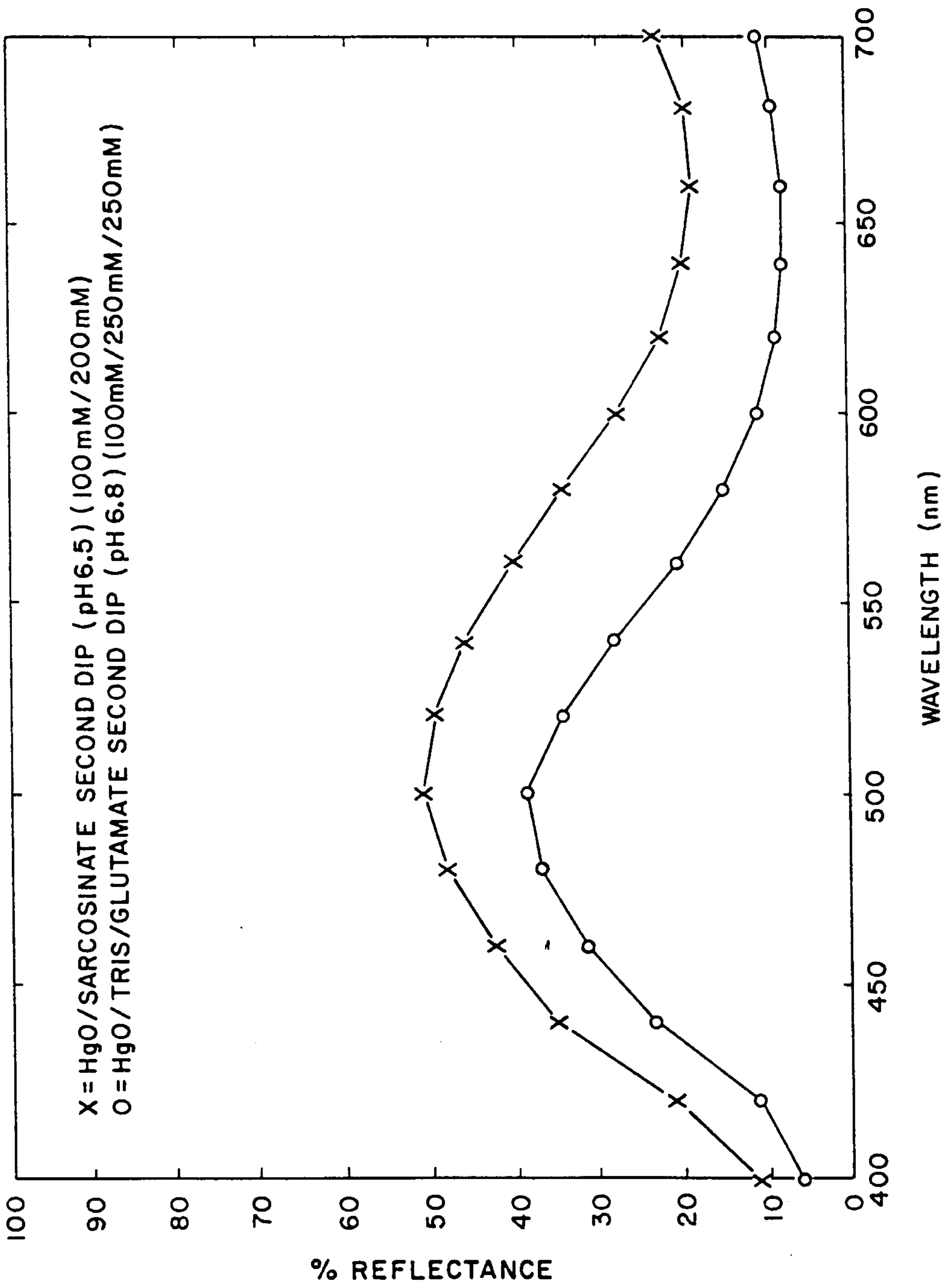
X = HgO/SARCOSINATE SECOND DIP (pH6.5) (100mM/200mM)
O = HgO/TRIS/GLUTAMATE SECOND DIP (pH 6.8) (100mM/250mM/250mM)
% REFLECTANCE
0
10
20
30
40
50
60
70
80
90
100
WAVELENGTH (nm)
400
450
500
550
600
650
700

FIG. I

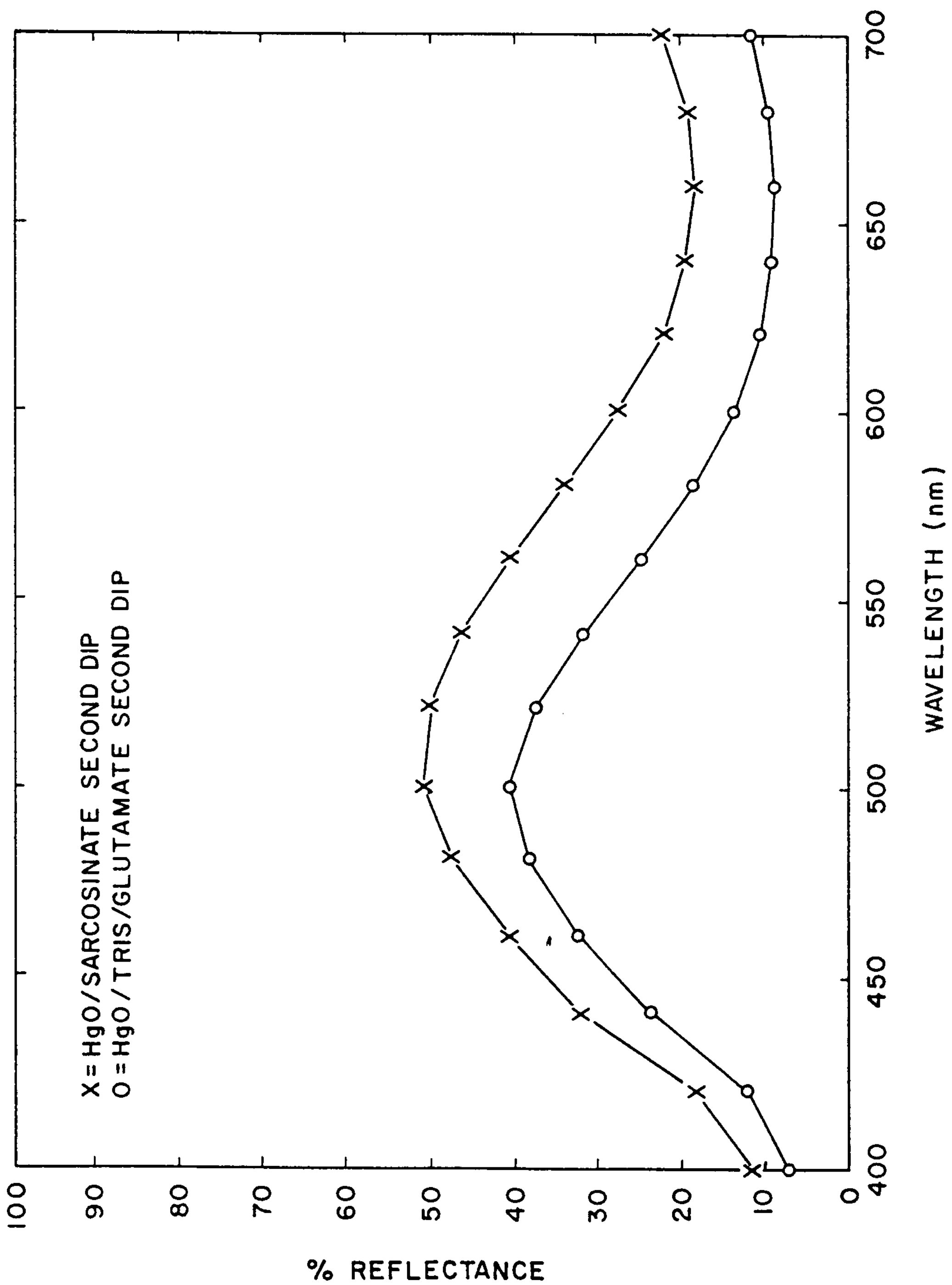
X = HgO/SARCOSINATE SECOND DIP
O = HgO/TRIS/GLUTAMATE SECOND DIP
% REFLECTANCE
100
90
80
70
60
50
40
30
20
10
0
WAVELENGTH (nm)
400
450
500
550
600
650
700

FIG. 2

ASCORBATE RESISTANCE OF STRIP
WITH TRIS/GLUTAMATE/HgO COMPLEX

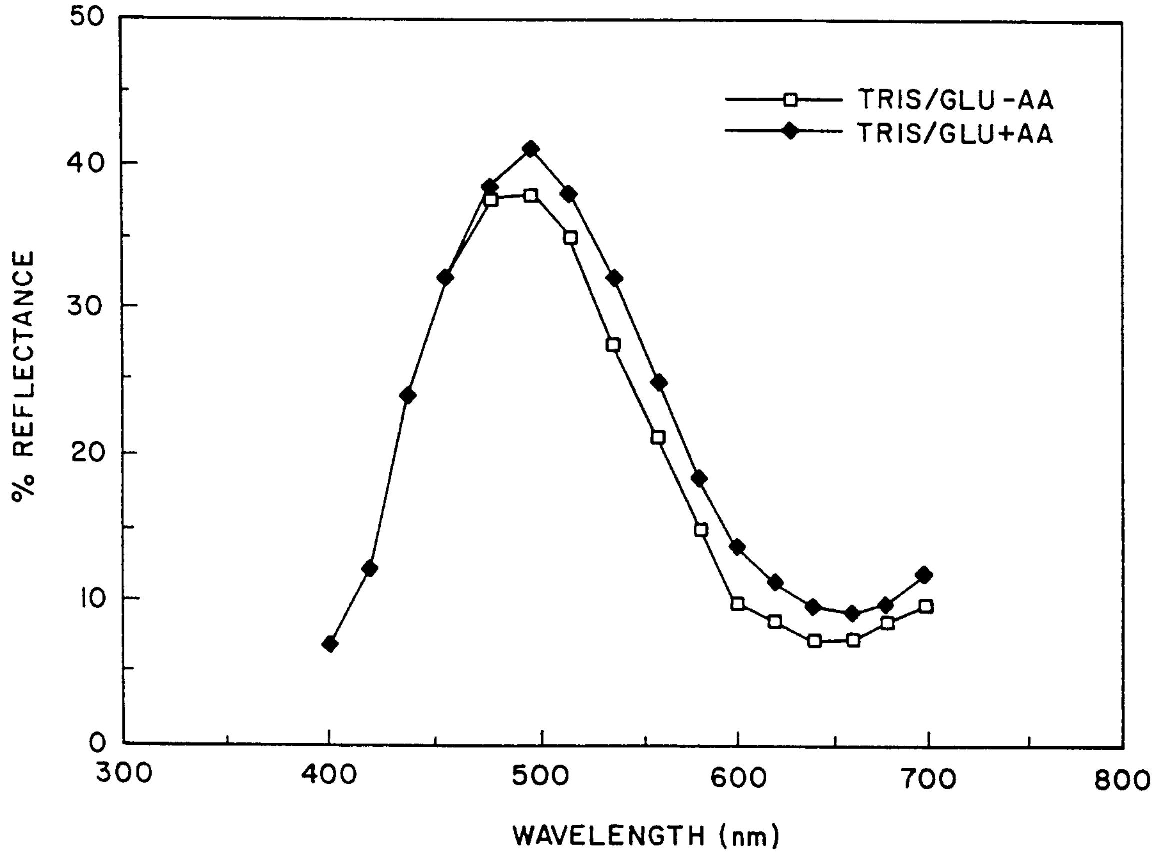


FIG. 3

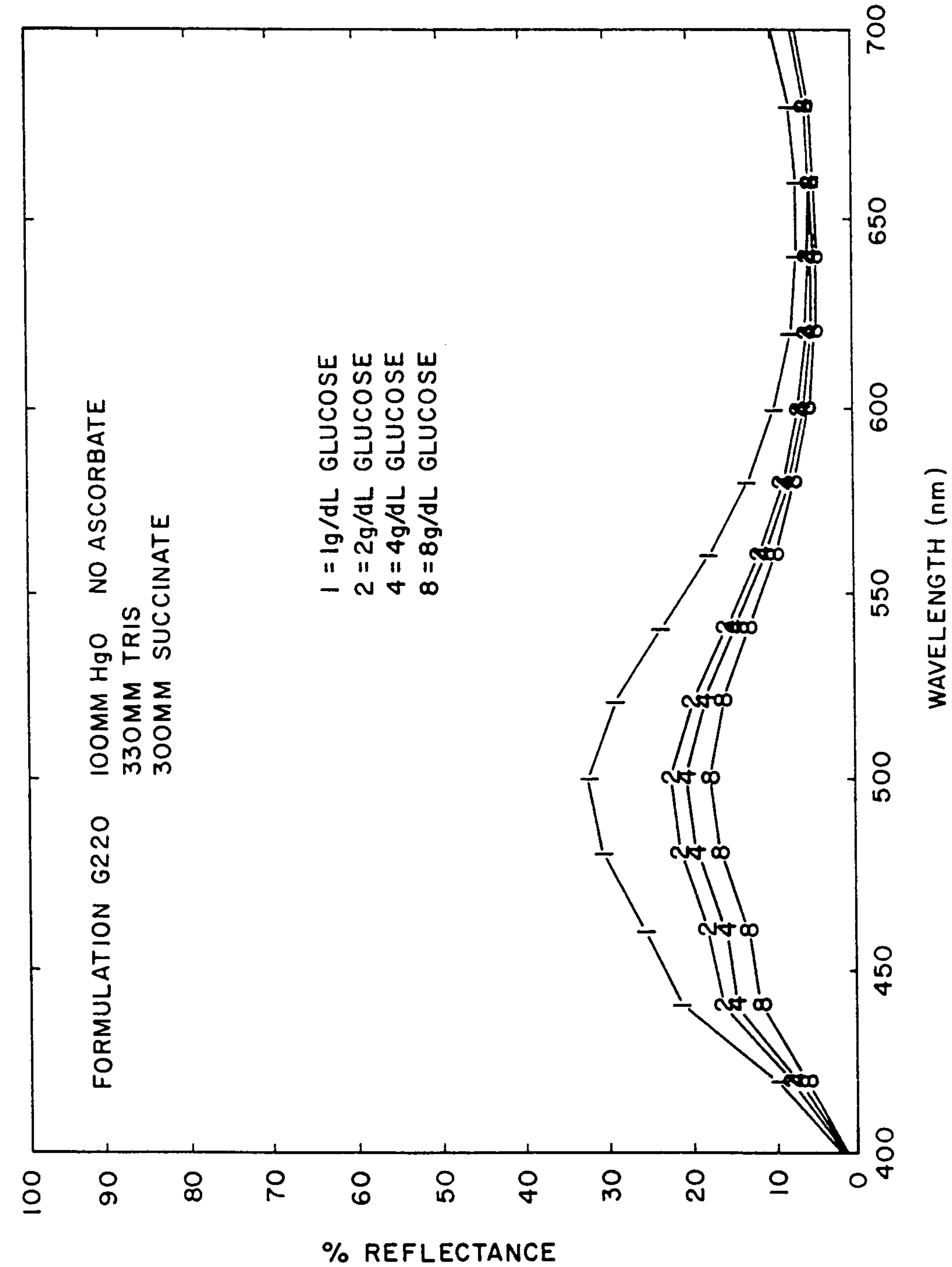
FORMULATION G220 100MM HgO NO ASCORBATE
330MM TRIS
300MM SUCCINATE
1 = 1g/dL GLUCOSE
2 = 2g/dL GLUCOSE
4 = 4g/dL GLUCOSE
8 = 8g/dL GLUCOSE
% REFLECTANCE
WAVELENGTH (nm)
0
10
20
30
40
50
60
70
80
90
100
400
450
500
550
600
650
700

FIG. 4

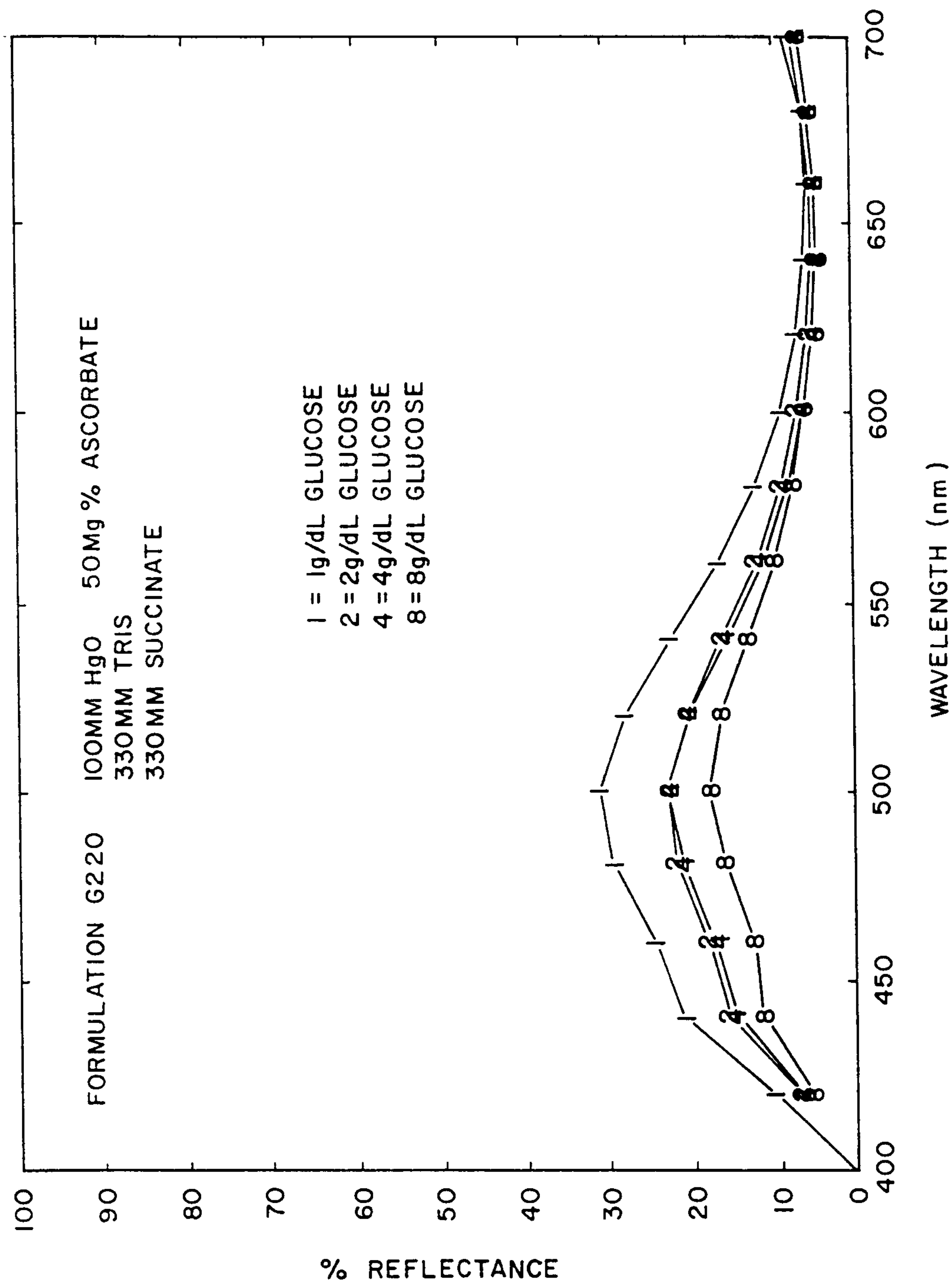
FORMULATION G220
100MM HgO
50Mg % ASCORBATE
330MM TRIS
330MM SUCCINATE
1 = 1g/dL GLUCOSE
2 = 2g/dL GLUCOSE
4 = 4g/dL GLUCOSE
8 = 8g/dL GLUCOSE
% REFLECTANCE
0
10
20
30
40
50
60
70
80
90
100
WAVELENGTH (nm)
400
450
500
550
600
650
700

FIG. 5

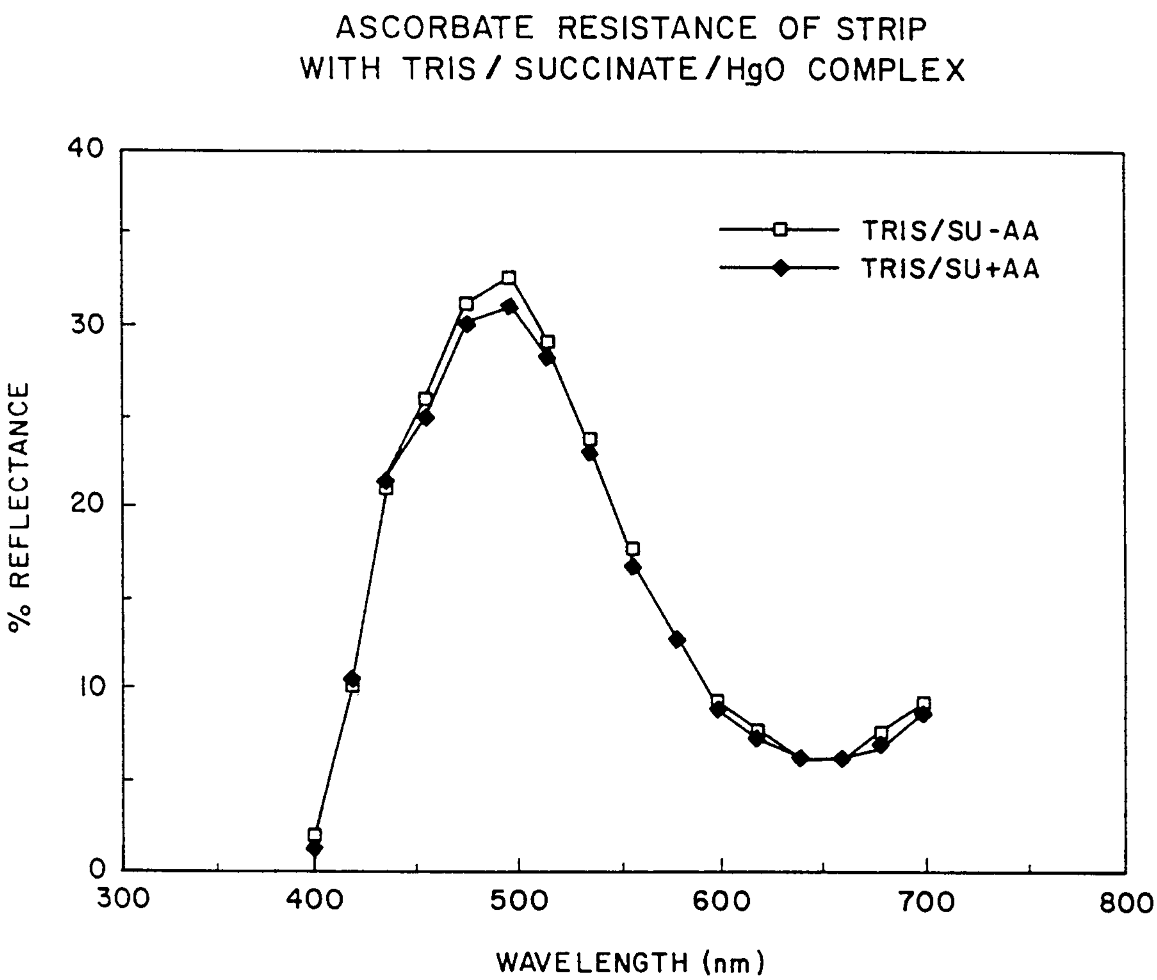
ASCORBATE RESISTANCE OF STRIP
WITH TRIS / SUCCINATE / HgO COMPLEX
TRIS/SU-AA
TRIS/SU+AA
% REFLECTANCE
0
10
20
30
40
300
400
500
600
700
800
WAVELENGTH (nm)

FIG. 6

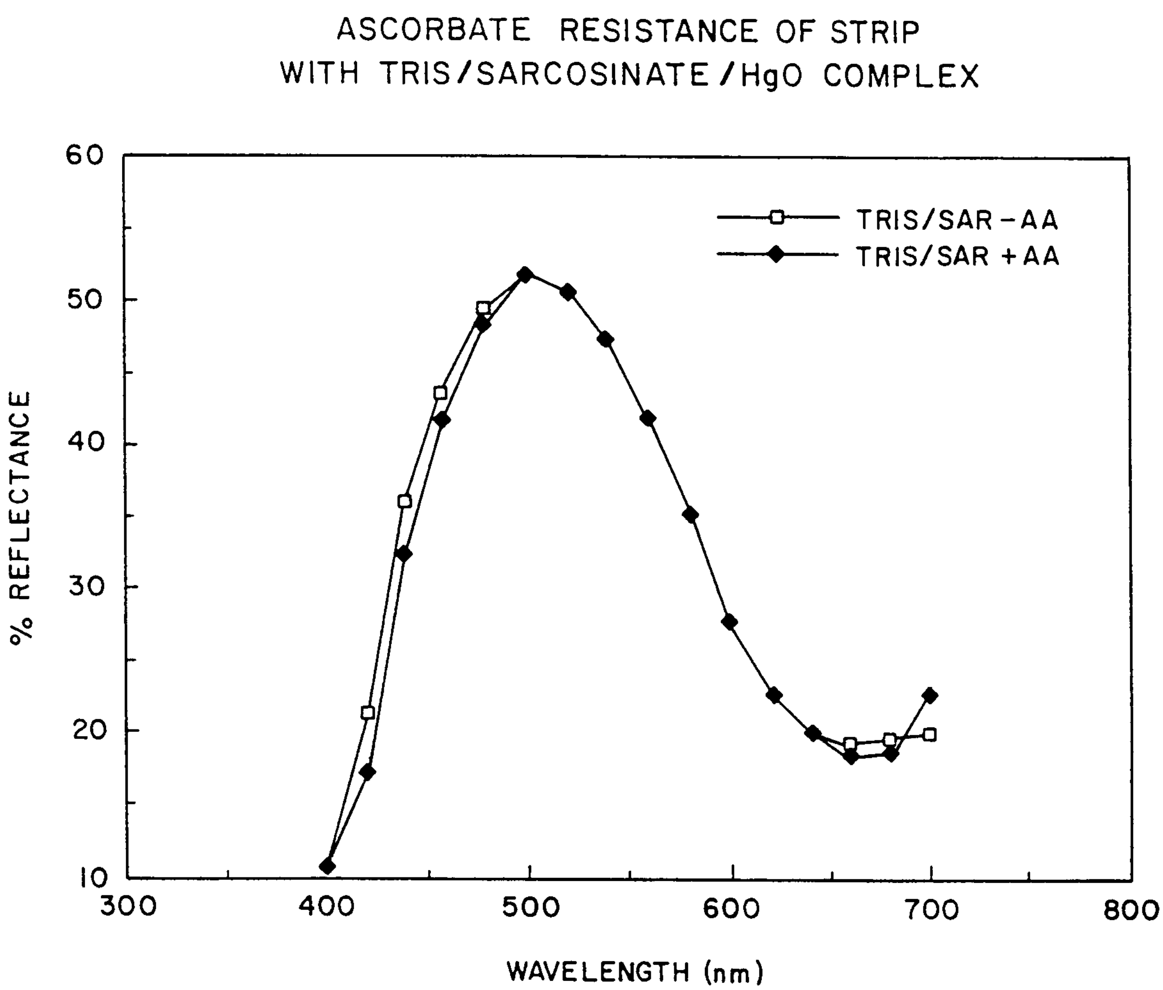
ASCORBATE RESISTANCE OF STRIP
WITH TRIS/SARCOSINATE/HgO COMPLEX
60
50
40
30
20
10
% REFLECTANCE
300
400
500
600
700
800
WAVELENGTH (nm)
TRIS/SAR -AA
TRIS/SAR +AA

FIG. 7